# EUROPEAN PATENT SPECIFICATION

(11) **EP 1 140 072 B1**
(45) Date of publication and mention of the grant of the patent: **14.04.2004**
(21) Application number: 99967619.0
(22) Date of filing: 28.12.1999
(51) Int. Cl.: A61K 31/41, A61K 31/36, A61K 31/135, A61K 31/165

(54) **COMPOUNDS AND METHODS**
ZUSAMMENSETZUNG UND VERFAHREN
COMPOSES ET PROCEDES

(30) Priority: 30.12.1998 US 114239 P; 06.04.1999 US 128010 P
(43) Date of publication of application: 10.10.2001
(73) Proprietor: SMITHKLINE BEECHAM CORPORATION, Philadelphia Pennsylvania 19101 (US)
(72) Inventor: BONDINELL, William, E., Wayne, PA 19087 (US); KU, Thomas, W., Dresher, PA 19025 (US); WANG, Ning, Phoenixville, PA 19460 (US)
(74) Representative: Blakey, Alison Jane
(86) International application number: PCT/US1999/030888
(87) International publication number: WO 2000/040239

(56) References cited:
- WO-A1-99/01127
- US-A- 4 091 097

## Description

### FIELD OF THE INVENTION

This invention relates to substituted benzanilides which are modulators, agonists or antagonists, of the CC chemokine receptor CC-CKR5 now designated as CCR5 (*Nature Medicine* **1996**, *2*, 1174-8). In addition, this invention relates to the use of these substituted benzanilides for the manufacture of a medicament for the treatment and prevention of disease states mediated by CCR5.

### BACKGROUND OF THE INVENTION

T cells are not only key regulators of the immune response to infectious agents but are believed critical for the initiation and maintenance of the inflammatory reaction in a variety of chronic diseases. Increased numbers or enhanced activation state of T cells, especially CD4+ T cells, have been demonstrated in the synovium of individuals with rheumatoid arthritis (M.J. Elliott and R. N. Maini, Int. Arch. Allergy Immunol. 104: 112-1125, 1994), in the bronchial mucosa of asthmatics (C.J. Corrigan and A.B. Kay, Immunol. Today 13:501-506, 1992), in the lesions of multiple sclerosis (R. Martin and H. F. McFarland, Crit. Rev. Clin. Lab. Sci. 32: 121-182, 1995), in psoriatic lesions (J.L. Jones, J. Berth-Jone, A. Fletcher and P.E. Hutchinson, J. Pathol. 174: 77-82, 1994) and in the fatty streaks of atherosclerosis (R. Ross, Annu. Rev. Physiol. 57: 791-804, 1995).

T cells, as well as other inflammatory cells, will migrate into tissues in response to the production of a variety chemotactic factors. Among these factors are a superfamily of 8-12 kDa proteins known as the chemokines. These proteins share structural features such as the presence of 3-4 conserved cysteine residues. RANTES, which stands for Regulated upon Activation Normal T cell Expressed and Secreted, is a 8 kDa protein member of CC branch of the chemokine family. These proteins recruit and activate immune and inflammatory cells through an interaction with G-protein coupled receptors. The CC branch is defined by the absence of an intervening amino acid residue between the first two cysteine residues and members of this family predominately elicit the migration of mononuclear cells, eosinophils and basophils (M. Baggiolini, B. Dewald, and B. Moser, Adv. Immunol. 55: 97-179, 1994; and J.J. Oppenheim, C.O.C. Zachariae, N. Mukaida, and K. Matsushima, Annu. Rev. Immunol. 9: 617-648, 1991).

RANTES potently produces chemotaxis of T cells, basophils, eosinophils, monocytes and mast cells. RANTES was originally identified as gene product induced late after antigen activation of T-cells (T.J. Schall, J. Jongstra, B.J. Dyer, J. Jorgensen, et al., J. Immunol. 141:1018-1025, 1988), however, RANTES has been shown to be synthesized and secreted by a diverse group of cells that include epithelial and endothelial cells (C. Stellato, L.A. Beck, G.A. Gorgone, D. Proud, et al., J. Immunol. 155: 410-418, 1995; and A. Marfaing-Koka, O. Devergne, G. Gorgone, A. Portier, et al., J. Immunol. 154: 1870-1878, 1994), synovial fibroblasts (P. Rathanaswami, M. Hachicha, M. Sadick, T.J. Schall, et al., J. Biol. Chem. 268: 5834-5839, 1993) and dermal fibroblasts (M. Sticherling, M. Kupper, F. Koltrowitz, E. Bomscheuer, et al., J. Invest. Dermatol. 105: 585-591, (1995), mesangial cells (G. Wolf, S. Aberle, F. Thaiss, et al., Kidney Int. 44: 795-804, 1994) and platelets (Y. Koameyoshi, A. Dorschner, A.I. Mallet, E. Christophers, et al., J. Exp. Med. 176: 587-592, 1992). In these cells RANTES mRNA is rapidly upregulated in response to IL-1 or TNFa. Although RANTES mRNA is not usually detected in normal tissues (J.M. Pattison, P.J. Nelson, and A.M. Krensky, Clin. Immunother. 4: 1-8, 1995), increased mRNA or protein has been found in diseases characterized by a mononuclear infiltrate. For example, RANTES mRNA was visualized using *in situ* hybridization in renal allografts undergoing rejection (J.M. Pattison, P.J. Nelson, and A.M. Krensky, Clin. Immunother. 4: 1-8, 1995; and K.C. Nadeau, H. Azuma and N.I. Tilney, Proc. Natl. Acad. USA 92: 8729-8733, 1995) in the skin of atopic dermatitis patients after exposure to antigen (S. Ying, L. Taborda-Barata, Q. Meng, M. Humbert, et al., J. Exp. Med. 181: 2153-2159, 1995), and in endothelial cells of coronary arteries undergoing accelerated atherosclerosis after cardiac transplant (J.M. Pattison, P.J. Nelson, and A.M. Krensky, Clin. Immunother. 4: 1-8, 1995). Further, increased immunoreactive protein for RANTES has been detected in bronchoalveolar lavage fluid (R. Alam, J. York, M. Boyers, et al., Am. J. Resp. Crit. Care Med. 149: A951, 1994) and sputum from asthmatic individuals (C.M. Gelder, P.S. Thomas, D.H. Yates, I.M. Adcock, et al., Thorax 50: 1033-1037, 1995).

Several receptors have been identified that bind RANTES. In particular, CCR5, when expressed in either HEK 293 cells or CHO cells, binds RANTES. This receptor is expressed in T-cells and in monocytes and macrophages, immune/inflammatory cells which are important in the maintenance of a chronic inflammatory reaction. Pharmacological characterization of CCR5 indicates similarities to the RANTES binding site observed on isolated T cells. Therefore, antagonism of RANTES' action on CCR5, as well as antagonism of other natural modulators of CCR5, should inhibit the recruitment of T cells and macrophages into inflammatory lesions and provide a novel therapeutic approach for the treatment of atopic and autoimmune disorders.

Since T cells express CCR5, selective receptor modulators of CCR5, particularly antagonists, are likely to provide beneficial effects in diseases like, asthma and atopic disorders (for example, atopic dermatitis and allergies), rheumatoid arthritis, sarcoidosis and other fibrotic diseases, atherosclerosis, psoriasis, autoimmune diseases such as multiple sclerosis, and inflammatory bowel disease, all in mammals, preferably humans. Furthermore, since CD8+ T cells have been implicated in chronic obstructive pulmonary disorders (COPD), CCR5 may play a role in their recruitment and therefore antagonists to CCR5 could provide potential therapeutic in the treatment of COPD. Also, since CCR5 is a co-receptor for the entry of HIV into cells, selective receptor modulators may be useful in the treatment of HIV infection.

A subset of compounds included in formula (I) have been reported to have 5-HT receptor activity (international application publication number WO 95/15954, published 15 June 1995, international application publication number WO 95/17398, published 29 June 1995, international application publication number WO 95/26328, published 5 October 1995, international application publication number WO 96/06079, published 29 February 1996, GB 2276161 published 21 September 1994, and GB 2276165 published 21 September 1994; international application publication number WO 95/30675 published 16 November 1995;international application publication number WO 95/17401 published 29 June 1995;international application publication number WO 96/31508 published 10 October 1996; international application publication number WO 97/10824 published 27 March 1997; international application publication number WO 96/11934 published 25 April 1996; international application publication number WO 96/19477 published 27 June 1996; international application publication number WO 97/17350 published 15 May 1997; international application publication number WO 97/34900 published 25 September 1997; international application publication number WO 97/34901 published 25 September 1997; international application publication number WO 97/35862 published 2 October 1997; international application publication number WO 97/19070 published 29 May 1997; international application publication number WO 95/32967 published 7 December 1995; international application publication number WO 97/07120 published 27 February 1997; U.S. Patent 3,931,195, issued Jan. 6, 1976; and U.S. Patent 4,000,143, issued Dec. 28, 1976). In addition, WO 99/01127, published January 14, 1999, discloses substituted benzanilides useful for modulating CCR5-mediated diseases.

Surprisingly, it has now been discovered that this class of non-peptide compounds, in particular substituted benzanilides of formula (I), function as CCR5 receptor modulators, and therefore, have utility in the treatment and prevention of disease states mediated by CCR5 receptor mechanisms.

### SUMMARY OF THE INVENTION

The present invention is to compounds of formula (I) and their use as CCR5 modulators for the treatment of certain disease states, like COPD, asthma and atopic disorders (for example, atopic dermatitis and allergies), rheumatoid arthritis, sarcoidosis and other fibrotic diseases, atherosclerosis, psoriasis, autoimmune diseases such as multiple sclerosis, inflammatory bowel disease, and HIV infection, all in mammals, preferably humans. Compounds of formula (I) have been described in copending patent application Attorney Docket No. P50680, provisional application serial number 60/051,632, filed July 3, 1997, published as international application WO 99/01127 on January 14, 1999. The preferred compounds for use as CCR5 modulators herein are those compounds of formula (I) as noted.

### DETAILED DESCRIPTION OF THE INVENTION

It has now been discovered that substituted benzanilides of formula (I) are particularly potent CCR5 receptor modulators. Selective inhibition of CCR5 receptor mechanisms by treatment with the receptor modulators of formula (I), or a pharmaceutically acceptable salt thereof, represents an effective therapeutic and preventative approach to the treatment of a variety of disease states, including asthma and atopic disorders (for example, atopic dermatitis and allergies), rheumatoid arthritis, sarcoidosis and other fibrotic diseases, atherosclerosis, psoriasis, autoimmune diseases such as multiple sclerosis, and inflammatory bowel disease, all in mammals, preferably humans. Furthermore, since CD8+ T cells have been implicated in COPD, CCR5 may play a role in their recruitment and therefore antagonists to CCR5 could provide potential therapeutic in the treatment of COPD. Also, since CCR5 is a co-receptor for entry into cells, selective receptor modulators may be useful in the treatment of HIV infection.

Compounds for use herein as CCR5 modulators include the 5-HT ligands as described in international application publication number WO 95/15954, published 15 June 1995, (USSN 08/652,581, filed June 7, 1996); international application publication number WO 95/17398, published 29 June 1995, (USSN 08/663,291, filed June 21, 1996); international application publication number WO 95/26328, published 5 October 1995, (USSN 08/718,481, filed Sept. 26, 1996); international application publication number WO 96/06079, published 29 February 1996, (USSN 08/793,428, filed Feb. 21, 1997); GB 2276161 published 21 September 1994, and GB 2276165 published 21 September 1994; international application publication number WO 95/30675, published 16 November 1995, (USSN 08/737,147); international application publication number WO 95/17401, published 29 June 1995 (USSN 08/663,290, filed June 21, 1996); international application publication number WO 96/31508, published 10 October 1996 (USSN 08/930,848, filed Oct. 7, 1997); international application publication number WO 97/10824, published 27 March 1997 (USSN 09/043,346); international application publication number WO 96/11934, published 25 April 1996, (USSN 08/817,619); international application publication number WO 96/19477, published 27 June 1996 (USSN 08/849,932); international application publication number WO 97/17350, published 15 May 1997 (USSN 09/068,382, filed May 8, 1998); international application publication number WO 97/34900, published 25 September 1997; international application publication number WO 97/34901, published 25 September 1997; international application publication number WO 97/35862, published 2 October 1997; international application publication number WO 97/19070, published 29 May 1997 (USSN 09/077,263); international application publication number WO 95/32967, published 7 December 1995 (USSN 08/737,660); international application publication number WO 97/07120, published 27 February 1997 (USSN 09/011,338, filed Feb. 11, 1998); U.S. Patent 3,931,195, issued Jan. 6, 1976; and U.S. Patent 4,000,143, issued Dec. 28, 1976.

The compounds of the invention can exist in unsolvated as well as solvated forms, including hydrated forms. In general, the solvated forms, with pharmaceutically acceptable solvents such as water, ethanol, and the like, are equivalent to the unsolvated forms for purposes of this invention.

The compounds of the present invention may contain one or more asymmetric carbon atoms and may exist in racemic and optically active forms. The stereocenters may be of any combination of R and S configuration, for example, (R,R), (R,S), (S,S) or (S,R). All of these compounds are within the scope of the present invention.

Among the preferred compounds of the invention are the following compounds:
N-[3-[2-[Bis(1-methylethyl)amino]ethoxy]-4-methoxyphenyl]-2'-ethyl-1,1'-biphenyl-4-carboxamide;
N-[3-[2-[Bis(1-methylethyl)amino]ethoxy]-4-methoxyphenyl]-2'-methoxy-1,1'-biphenyl-4-carboxamide:
N-[3-[2-[Bis(1-methylethyl)amino]ethoxy]-4-methoxyphenyl]-2',6'-dimethyl-1,1'-biphenyl-4-carboxamide;
N-[3-[2-[Bis(1-methylethyl)amino]ethoxy]-4-methoxyphenyl]-3'-ethoxycarbonyl-1,1'-biphenyl-4-carboxamide;
N-[3-[2-[Bis(1-methylethyl)amino]ethoxy]-4-methoxyphenyl]-2',6'-dichloro-1,1'-biphenyl-4-carboxamide.;
N-[3-[2-[Bis(1-methylethyl)amino]ethoxy]-4-methoxyphenyl]-2'-chloro-1,1'-biphenyl-4-carboxamide;
N-[3-[2-[Bis(1-methylethyl)amino]ethoxy]-4-methoxyphenyl]-2',4'-dichloro-1,1'-bipheyl-4-carboxamide;
N-[3-[2-[Bis(1-methylethyl)amino]ethoxy]-4-methoxyphenyl]-3'-acetyl-1,1'-biphenyl-4-carboxamide trifluoroacetate;
N-[3-[2-[Bis(1-methylethyl)amino]ethoxy]-4-methoxyphenyl]-3'-methylthio-1,1'-biphenyl-4-carboxamide;
N-[3-[2-[Bis(1-methylethyl)amino]ethoxy]-4-methoxyphenyl]-3',5'-dichloro-1,1'-biphenyl-4-carboxamide;
N-[3-[2-[Bis(1-methylethyl)amino]ethoxy]-4-methoxyphenyl]-3'-isopropoxycarbonyl-1,1'-biphenyl-4-carboxamide;
N-[3-[2-[Bis(1-methylethyl)amino]ethoxy]-4-methoxyphenyl]-3',5'-bis(methoxycarbonyl)-1,1'-biphenyl-4-carboxamide;
N-[3-[2-[Bis(1-methylethyl)amino]ethoxy]-4-methoxyphenyl]-3'-propionyl-1,1'-biphenyl-4-carboxamide trifluoroacetate;
N-[3-[2-[Bis(1-methylethyl)amino]ethoxy]-4-methoxyphenyl]-3'-propoxy-1,1'-biphenyl-4-carboxamide;
N-[3-[2-[Bis(1-methylethyl)amino]ethoxy]-4-methoxyphenyl]-3'-methoxycarbonyl-1,1'-biphenyl-4-carboxamide trifluoroacetate;
N-[3-[2-[Bis(1-methylethyl)amino]ethoxy]-4-methoxyphenyl]-3'-methyl-1,1'-biphenyl-4-carboxamide trifluoroacetate; and
N-[3-[2-[Bis(1-methylethyl)amino]ethoxy]-4-methoxyphenyl]-2',3'-dimethyl-1,1'-biphenyl-4-carboxamide trifluoroacetate.

Among the more preferred compounds of the invention is the following compound:
N-[3-[2-[Bis(1-methylethyl)amino]ethoxy]-4-methoxyphenyl]-3'-ethoxycarbonyl-1,1'-biphenyl-4-carboxamide;
N-[3-[2-[Bis(1-methylethyl)amino]ethoxy]-4-methoxyphenyl]-3',5'-dichloro-1,1'-biphenyl-4-carboxamide;
N-[3-[2-(Bis(1-methylethyl)amino]ethoxy]-4-methoxyphenyl]-3'-propoxy-1,1'-biphenyl-4-carboxamide;
N-[3-[2-[Bis(1-methylethyl)amino]ethoxy]-4-methoxyphenyl]-3'-methyl-1,1'-biphenyl-4-carboxamide trifluoroacetate; and
N-[3-[2-[Bis(1-methylethyl)amino]ethoxy]-4-methoxyphenyl]-2',3'-dimethyl-1,1'-biphenyl-4-carboxamide trifluoroacetate.

### Formulation of Pharmaceutical Compositions

The pharmaceutically effective compounds of this invention (and the pharmaceutically acceptable salts thereof) are administered in conventional dosage forms prepared by combining a compound of formula (I) ("active ingredient") in an amount sufficient to treat COPD, asthma and atopic disorders (for example, atopic dermatitis and allergies), rheumatoid arthritis, sarcoidosis and other fibrotic diseases, atherosclerosis, psoriasis, autoimmune diseases such as multiple sclerosis, inflammatory bowel disease, and HIV infection, ("CCR5-mediated disease states") with standard pharmaceutical carriers or diluents according to conventional procedures well known in the art. These procedures may involve mixing, granulating and compressing or dissolving the ingredients as appropriate to the desired preparation.

The pharmaceutical carrier employed may be, for example, either a solid or liquid. Exemplary of solid carriers are lactose, terra alba, sucrose, talc, gelatin, agar, pectin, acacia, magnesium stearate, stearic acid and the like. Exemplary of liquid carriers are syrup, peanut oil, olive oil, water and the like. Similarly, the carrier or diluent may include time delay material well known to the art, such as glyceryl monostearate or glyceryl distearate alone or with a wax.

A wide variety of pharmaceutical forms can be employed. Thus, if a solid carrier is used, the preparation can be tableted, placed in a hard gelatin capsule in powder or pellet form or in the form of a troche or lozenge. The amount of solid carrier will vary widely but preferably will be from about 25 mg to about 1000 mg. When a liquid carrier is used, the preparation will be in the form of a syrup, emulsion, soft gelatin capsule, sterile injectable liquid such as an ampule or nonaqueous liquid suspension.

The active ingredient may also be administered topically to a mammal in need of treatment or prophylaxis of CCR5 mediated disease states. The amount of active ingredient required for therapeutic effect on topical administration will, of course, vary with the compound chosen, the nature and severity of the disease state being treated and the mammal undergoing treatment, and is ultimately at the discretion of the physician. A suitable dose of an active ingredient is 1.5 mg to 500 mg for topical administration, the most preferred dosage being 1 mg to 100 mg, for example 5 to 25 mg administered two or three times daily.

By topical administration is meant non-systemic administration and includes the application of the active ingredient externally to the epidermis, to the buccal cavity and instillation of such a compound into the ear, eye and nose, and where the compound does not significantly enter the blood stream. By systemic administration is meant oral, intravenous, intraperitoneal and intramuscular administration.

While it is possible for an active ingredient to be administered alone as the raw chemical, it is preferable to present it as a pharmaceutical formulation. The active ingredient may comprise, for topical administration, from 0.001% to 10% w/w, e.g. from 1% to 2% by weight of the formulation although it may comprise as much as 10% w/w but preferably not in excess of 5% w/w and more preferably from 0.1% to 1% w/w of the formulation.

The topical formulations of the present invention, both for veterinary and for human medical use, comprise an active ingredient together with one or more acceptable carrier(s) therefor and optionally any other therapeutic ingredient(s). The carrier(s) must be 'acceptable' in the sense of being compatible with the other ingredients of the formulation and not deleterious to the recipient thereof.

Formulations suitable for topical administration include liquid or semi-liquid preparations suitable for penetration through the skin to the site of inflammation such as liniments, lotions, creams, ointments or pastes, and drops suitable for administration to the eye, ear or nose.

Drops according to the present invention may comprise sterile aqueous or oily solutions or suspensions and may be prepared by dissolving the active ingredient in a suitable aqueous or alcoholic solution of a bactericidal and/or fungicidal agent and/or any other suitable preservative, and preferably including a surface active agent. The resulting solution may then be clarified by filtration, transferred to a suitable container which is then sealed and sterilized by autoclaving or maintaining at 98-100°C for half an hour. Alternatively, the solution may be sterilized by filtration and transferred to the container by an aseptic technique. Examples of bactericidal and fungicidal agents suitable for inclusion in the drops are phenylmercuric nitrate or acetate (0.002%), benzalkonium chloride (0.01 %) and chlorhexidine acetate (0.01 %). Suitable solvents for the preparation of an oily solution include glycerol, diluted alcohol and propylene glycol.

Lotions according to the present invention include those suitable for application to the skin or eye. An eye lotion may comprise a sterile aqueous solution optionally containing a bactericide and may be prepared by methods similar to those for the preparation of drops. Lotions or liniments for application to the skin may also include an agent to hasten drying and to cool the skin, such as an alcohol or acetone, and/or a moisturizer such as glycerol or an oil such as castor oil or arachis oil.

Creams, ointments or pastes according to the present invention are semi-solid formulations of the active ingredient for external application. They may be made by mixing the active ingredient in finely-divided or powdered form, alone or in solution or suspension in an aqueous or non-aqueous fluid, with the aid of suitable machinery, with a greasy or non-greasy basis. The basis may comprise hydrocarbons such as hard, soft or liquid paraffin, glycerol, beeswax, a metallic soap; a mucilage; an oil of natural origin such as almond, corn, arachis, castor or olive oil; wool fat or its derivatives, or a fatty acid such as stearic or oleic acid together with an alcohol such as propylene glycol. The formulation may incorporate any suitable surface active agent such as an anionic, cationic or non-ionic surfactant such as esters or polyoxyethylene derivatives thereof. Suspending agents such as natural gums, cellulose derivatives or inorganic materials such as silicaceous silicas, and other ingredients such as lanolin, may also be included.

The active ingredient may also be administered by inhalation. By "inhalation" is meant intranasal and oral inhalation administration. Appropriate dosage forms for such administration, such as an aerosol formulation or a metered dose inhaler, may be prepared by conventional techniques. The daily dosage amount of the active ingredient administered by inhalation is from about 0.1 mg to about 100 mg per day, preferably about 1 mg to about 10 mg per day.

In one aspect, this invention relates to a method of treating COPD, asthma and atopic disorders (for example, atopic dermatitis and allergies), rheumatoid arthritis, sarcoidosis and other fibrotic diseases, atherosclerosis, psoriasis, autoimmune diseases such as multiple sclerosis, inflammatory bowel disease, and HIV infection, all in mammals, preferably humans, which comprises administering to such mammal an effective amount of a CCR5 receptor modulator.

By the term "treating" is meant either prophylactic or therapeutic therapy. Such compound can be administered to such mammal in a conventional dosage form prepared by combining the compound with a conventional pharmaceutically acceptable carrier or diluent according to known techniques. It will be recognized by one of skill in the art that the form and character of the pharmaceutically acceptable carrier or diluent is dictated by the amount of active ingredient with which it is to be combined, the route of administration and other well-known variables. The compound is administered to a mammal in need of treatment for asthma and atopic disorders (for example, atopic dermatitis and allergies), rheumatoid arthritis, atherosclerosis, psoriasis, autoimmune diseases such as multiple sclerosis, inflammatory bowel disease, and HIV infection, in an amount sufficient to decrease symptoms associated with these disease states. The route of administration may be oral or parenteral.

The term parenteral as used herein includes intravenous, intramuscular, subcutaneous, intra-rectal, intravaginal or intraperitoneal administration. The subcutaneous and intramuscular forms of parenteral administration are generally preferred. The daily parenteral dosage regimen will preferably be from about 30 mg to about 300 mg per day of active ingredient. The daily oral dosage regimen will preferably be from about 100 mg to about 2000 mg per day of active ingredient.

It will be recognized by one of skill in the art that the optimal quantity and spacing of individual dosages of the compound will be determined by the nature and extent of the condition being treated, the form, route and site of administration, and the particular mammal being treated, and that such optimums can be determined by conventional techniques. It will also be appreciated by one of skill in the art that the optimal course of treatment, i.e., the number of doses of the compound given per day for a defined number of days, can be ascertained by those skilled in the art using conventional course of treatment determination tests.

### Methods of Preparation

The compounds can be prepared by art-recognized procedures from known or commercially available starting materials. If the starting materials are unavailable from a commercial source, their synthesis is described herein, or they can be prepared by procedures known in the art, e.g. as described in WO95/17401.

The invention will now be described by reference to the following examples which are merely illustrative. In the Examples, mass spectra were performed upon a VG Zab mass spectrometer using fast atom bombardment, unless otherwise indicated.

### EXAMPLES

### Preparation 1

### Preparation of N-[3-[2-[Bis(1-methylethyl)amino]ethoxy]-4-methoxyphenyl]-4-iodobenzamide

A mixture of 4-iodobenzoyl chloride, prepared from 4-iodobenzoic acid (5.5 g, 22 mmol) and thionyl chloride (20 mL) at 70°C for 30 min followed by removal of thionyl chloride *in vacuo*, 3-(2-diisopropylamino)ethoxy-4-methoxyaniline (WO 95/15954) (5 g, 19 mmol), and diisopropylethylamine (4.9 g, 38 mmol) in dichloromethane (50 mL), was stirred at room temperature for 16 h. The mixture was diluted with dichloromethane, washed with 5% sodium carbonate and with water, dried (MgSO₄), and concentrated *in vacuo*. The resulting solid was treated with acetonitrile, filtered, washed with acetonitrile, and dried to give the title compound (8.15 g).

### Preparation 2

### Preparation of 2',6'-Dichloro-4-biphenylcarboxylic acid

A mixture of 2,6-dichloro-1-iodobenzene (0.5 g, 1.8 mmol), 4-carboxybenzeneboronic acid (0.3 g, 1.8 mmol), tetrakis(triphenylphosphine)-palladium(0) (40 mg), and sodium carbonate (0.68 g, 6.4 mmol) in a 1:1 mixture of 1,2-dimethoxyethane and water (26 mL) was heated at reflux for 16 h. The mixture was cooled and extracted with ether. The aqueous phase was acidified with 3M hydrochloric acid, allowed to stand for 16 h, and filtered. The filter cake was washed with water and dried to give the title compound.

### Preparation 3-11

### Preparation of 2'-Chloro-4-biphenylcarboxylic Acid; 2',4'-Dichloro-4-biphenylcarboxylic Acid; 3'-Methylthio-4-biphenylcarboxylic Acid; 3',5'-Dichloro-4-biphenylcarboxylic Acid; 3'-Isopropoxycarbonyl-4-biphenylcarboxylic Acid; 3'-Propionyl-4-biphenylcarboxylic Acid; 3'-Propoxy-4-biphenylcarboxylic Acid, 3'-Methyl-4-biphenylcarboxylic Acid, and 2',3'-Diethyl-4-biphenylcarboxylic Acid

Following the procedure of Preparation 2, except substituting 2-chloro-1-iodobenzene, 2,4-dichloro-1-iodobenzene, 3-methylthio-1-bromobenzene, 3,5-dichloro-1-iodobenzene, 3-isopropoxycarbonyl-1-iodobenzene, 3-propionyl-1-bromobenzene, 3-propoxy-1-bromobenzene, 3-methyl-1-bromobenzene, or 2,3-dimethyl-1-bromobenzene for 2,6-dichloro-1-iodobenzene, gave the title compounds:
2'-chloro-4-biphenylcarboxylic acid: mp 282-285°C;
2',4'-dichloro-4-biphenylcarboxylic acid;
3'-methylthio-4-biphenylcarboxylic acid;
3',5'-dichloro-4-biphenylcarboxylic acid;
3'-isopropoxycarbonyl-4-biphenylcarboxylic acid;
3'-propionyl-4-biphenylcarboxylic acid;
3'-propoxy-4-biphenylcarboxylic acid;
3'-methyl-4-biphenylcarboxylic acid; and
2',3'-dimethyl-4-biphenylcarboxylic acid.

### Example 1

### Preparation of N-[3-[2-[Bis(1-methylethyl)amino]ethoxy]-4-methoxyphenyl]-3'-ethoxycarbonyl-1,1'-biphenyl-4-carboxamide

A solution of 3-(ethoxycarbonyl)phenylzinc iodide in tetrahydrofuran (0.5M, 10 mL, 5 mmol) was added to a solution of the compound of Preparation 1 (1 g, 2 mmol) and tetrakis(triphenylphosphine)palladium(0) (0.2 g) in tetrahydrofuran (10 mL) and the mixture was stirred at RT for 16 h. The mixture was treated with saturated aqueous ammonium chloride, extracted with ether, and the combined organic phase was washed with water, dried (MgSO₄), and concentrated *in vacuo*. The residue was chromatographed (silica gel, 5% methanol/dichloromethane) to give the title compound (0.14 g): MS(ES) m/e 518.8 [M+H]⁺.

### Example 2-4

### Preparation of N-[3-[2-[Bis(1-methylethyl)amino]ethoxy]-4-methoxyphenyl]-2'-ethyl-1,1'-biphenyl-4-carboxamide, N-[3-[2-[Bis(1-methylethyl)amino]ethoxy]-4-methoxyphenyl]-2'-methoxy-1,1'-biphenyl-4-carboxamide, and N-[3-[2-[Bis(1-methylethyl)amino]ethoxy]-4-methoxyphenyl]-2',6'-dimethyl-1,1'-biphenyl-4-carboxamide

Following the procedure of Example 1, except substituting 2-ethylphenylzinc iodide, 2-methoxyphenylzinc iodide, or 2,6-dimethylphenylzinc iodide for 3-(ethoxycarbonyl)phenylzinc iodide, gave the title compounds:
N-[3-[2-[bis(1-methylethyl)amino]ethoxy]-4-methoxyphenyl]-2'-ethyl-1,1'-biphenyl-4-carboxamide: MS(ES) m/e 474.9 [M+H]⁺;
N-[3-[2-[bis(1-methylethyl)amino]ethoxy]-4-methoxyphenyl]-2'-methoxy-1,1'-biphenyl-4-carboxamide: MS(ES) m/e 476.9 [M+H]⁺; and
N-[3-[2-[bis(1-methylethyl)amino]ethoxy]-4-methoxyphenyl]-2',6'-dimethyl-1,1'-biphenyl-4-carboxamide: MS(ES) m/e 474.8 [M+H]⁺.

### Example 5

### Preparation of N-[3-[2-[Bis(1-methylethyl)amino]ethoxy]-4-methoxyphenyl]-2',6'-dichloro-1,1'-biphenyl-4-carboxamide

The compound of Preparation 2 (0.25 g, 0.9 mmol), 3-(2-diisopropylamino)ethoxy-4-methoxyaniline (WO 95/15954)(0.26 g, 1 mmol), and benzotriazol-1-yloxy-tris(dimethylamino)phosphonium hexafluorophosphate (0.44 g, 1 mmol) was dissolved in acetonitrile (5 mL), and treated with triethylamine (0.2 g, 2 mmol). The mixture was stirred at RT for 16 h, treated with brine, and extracted with dichloromethane. The organic phase was washed with 5% sodium carbonate and with water, dried (MgSO₄), concentrated *in vacuo*, and purified by HPLC (ODS-A, 20 X 50 mm, A:acetonitrile B:water-0.1% trifluoroacetic acid, 10-90% during 10 min, UV detection at 254 nm) to give the title compound: MS(ES) m/e 514.7; 516.8 [M+H]⁺.

### Example 6-15

### Preparation of N-[3-[2-[Bis(1-methylethyl)amino]ethoxy]-4-methoxyphenyl]-2'-chloro-1,1'-biphenyl-4-carboxamide; N-[3-[2-[Bis(1-methylethyl)amino]ethoxy]-4-methoxyphenyl]-2',4'-dichloro-1,1'-biphenyl-4-carboxamide; N-[3-[2-[bis(1-methylethyl)amino]ethoxy]-4-methoxyphenyl]-3'-acetyl-1,1'-biphenyl-4-carboxamide trifluoroacetate; N-[3-[2-[Bis(1-methylethyl)amino]ethoxy]-4-methoxyphenyl]-3'-methylthio-1,1'-biphenyl-4-carboxamide; N-[3-[2-[Bis(1-methylethyl)amino]ethoxy]-4-methoxyphenyl]-3',5'-dichloro-1,1'-biphenyl-4-carboxamide; N-[3-[2-[Bis(1-methylethyl)amino]ethoxy]-4-methoxyphenyl]-3'-isopropoxycarbonyl-1,1'-biphenyl-4-carboxamideN-[3-[2-[Bis(1-methylethyl)amino]ethoxy]-4-methoxyphenyl]-3'-propionyl-1,1'-biphenyl-4-carboxamide trifluoroacetate; N-[3-[2-[Bis(1-methylethyl)amino]ethoxy]-4-methoxyphenyl]-3'-propoxy-1,1'-biphenyl-4-carboxamide; N-[3-[2-[Bis(1-methylethyl)amino]ethoxy]-4-methoxyphenyl]-3'-methyl-1,1'-biphenyl-4-carboxamide trifluoroacetate; and N-[3-[2-[Bis(1-methylethyl)amino]ethoxy]-4-methoxyphenyl]-2',3'-dimethyl-1,1'-biphenyl-4-carboxamide trifluoroacetate

Following the procedure of Example 5, except substituting the compound of Preparations 3 and 4, 3'-acetyl-4-biphenylcarboxylic acid (WO 9743262), or the compounds of Preparations 5-11 for the compound of Preparation 2, afforded the title compounds:
N-[3-[2-[bis(1-methylethyl)amino]ethoxy]-4-methoxyphenyl]-2'-chloro-1,1'-biphenyl-4-carboxamide: MS(ES) m/e 481.0 [M+H]⁺;
N-[3-[2-[bis(1-methylethyl)amino]ethoxy]-4-methoxyphenyl]-2',4'-dichloro-1,1'-bipheyl-4-carboxamide: MS(ES) m/e 515.1 [M+H]⁺;
N-[3-[2-[bis(1-methylethyl)amino]ethoxy]-4-methoxyphenyl]-3'-acetyl-1,1'-biphenyl-4-carboxamide trifluoroacetate: MS(ES) m/e 489.3 [M+H]⁺;
N-[3-[2-[bis(1-methylethyl)amino]ethoxy]-4-methoxyphenyl]-3'-methylthio-1,1'-biphenyl-4-carboxamide: MS(ES) m/e 493.3 [M+H]⁺;
N-[3-[2-[bis(1-methylethyl)amino]ethoxy]-4-methoxyphenyl]-3',5'-dichloro-1,1'-biphenyl-4-carboxamide: MS(ES) m/e 515.0 [M+H]⁺;
N-[3-[2-[bis(1-methylethyl)amino]ethoxy]-4-methoxyphenyl]-3'-isopropoxycarbonyl-1,1'-biphenyl-4-carboxamide: MS(ES) m/e 533.2 [M+H]⁺;
N-[3-[2-[Bis(1-methylethyl)amino]ethoxy]-4-methoxyphenyl]-3'-propionyl-1,1'-biphenyl-4-carboxamide trifluoroacetate: MS(ES) m/e 503.1 [M+H]⁺;
N-[3-[2-[Bis(1-methylethyl)amino]ethoxy]-4-methoxyphenyl]-3'-propoxy-1,1'-biphenyl-4-carboxamide: MS(ES) m/e 504.4 [M+H]⁺;
N-[3-[2-[Bis(1-methylethyl)amino]ethoxy]-4-methoxyphenyl]-3'-methyl-1,1'-biphenyl-4-carboxamide trifluoroacetate: MS(ES) m/e 461.2 [M+H]⁺; and
N-[3-[2-[Bis(1-methylethyl)amino]ethoxy]-4-methoxyphenyl]-2',3'-dimethyl-1,1'-biphenyl-4-carboxamide trifluoroacetate: MS(ES) m/e 475.2 [M+H]⁺.

### Example 16

### Preparation of N-[3-[2-[Bis(1-methylethyl)amino]ethoxy]-4-methoxyphenyl]-3',5'-bis(methoxycarbonyl)-1,1'-biphenyl-4-carboxamide

A suspension of the compound of Preparation 1 (0.5 g, 1 mmol) in tetrahydrofuran (5 mL) containing dichlorobis(triphenylphosphine)-palladium(II) (34 mg, 0.05 mmol) and 5-(tributylstannyl)-1,3-benzenedicarboxylic acid dimethyl ester [Cielen, et al, *J. Chem. Soc., Perkin Trans.* 2 (1998), 1573-1580] (0.48 g, 1 mmol) was stirred and heated to reflux for 40 h. Additional tetrahydrofuran was added and the mixture heated at reflux for 20 h, cooled, diluted with ether, and filtered through Celite™. The filtrate was diluted with ethyl acetate and decanted to remove insoluble material. The insoluble material was purified by HPLC (ODS-A, 20 X 50 mm, A:acetonitrile B:water-0.1% trifluoroacetic acid, 10-90% during 10 min, UV detection at 254 nm) to give the title compound: MS(ES) m/e 563.1 [M+H]⁺.

### Example 17

### Preparation of N-[3-[2-[Bis(1-methylethyl)amino]ethoxy]-4-methoxyphenyl]-3'-methoxycarbonyl-1,1'-biphenyl-4-carboxamide trifluoroacetate

A solution of the compound of Example 1 (0.13 g, 0.25 mmol) and sodium methoxide (0.01 g) in methanol (5 mL) was stirred at 65°C for 10 min, cooled, and concentrated *in vacuo*. The residue was dissolved in ethyl acetate, filtered, and the filtrate was dried (MgSO₄), concentrated *in vacuo*, and the residue was purified by HPLC (ODS-A, 20 X 50 mm, A:acetonitrile B:water-0.1% trifluoroacetic acid, 10-90% during 10 min, UV detection at 254 nm) to afford the title compound: MS(ES) m/e 505.0 [M+H]⁺.

### Biological Data:

### CCR5 Receptor Binding Assay

CHO cell membranes (0.25 x10⁶ cell equivalents) derived from CHO cells stably transfected with CCR5 were incubated with 0.3 ¹²⁵I-RANTES in a 96 well plate for 45 min. at room temperature (final reaction volume 200 ul). The reaction was terminated by filtration and the filters (GF/C) were washed twelve times with a solution of phosphate buffered saline containing 0.1 % bovine serum albumin and 0.05 % NaN₃. The radioactivity bound to filters was measured by liquid scintillation spectrometry. Non-specific binding was determined in the presence of unlabelled RANTES (10 or 30 nM) and averages 30-50% of total binding.

### CCR5 Receptor Functional Assay

The cellular functional assay used to assess antagonist activity of compounds was RANTES-induced Ca²⁺ mobilization in RBL 2H3 cells stably expressing the hCCR5 receptor (RBL 2H3 hCCR5). Agonist activity is determined by Ca²⁺ mobilization in the same cells which is inhibitable by a selective CCR5 antagonist. Cells were grown to 80-100% confluency in T-150 flasks and washed with phosphate-buffered saline. Cells were lifted from the flasks by treating with 3 mL of 1 mM EDTA for 3 min, at room temperature and diluting to 2 X 10⁶ cells/mL with Krebs Ringer Henseleit buffer (KRH; 118 mM NaCl, 4.6 mM KCI, 25 mM NaHCO₃, 1 mM KH₂PO₄ and 11 mM glucose) containing 5 mM HEPES (pH 7.4), 1 mM CaCl₂, 1 mM MgCl₂ and 0.1% BSA and centrifuged at 200g for 3 min. Cells were resuspended at 2 X 10⁶ cells/mL in the same buffer with 2 µM Fura-2AM, and incubated for 35 min. at 37°C. Cells were centrifuged at 200 x g for 3 min. and resuspended in the same buffer without Fura-2AM, then incubated for 15 min. at 37° C to complete the hydrolysis of intracellular Fura-2AM, and then centrifuged as before. Cells (10⁶ cells/mL) were resuspended in cold KRH with 5 mM HEPES (pH 7.4), 1 mM CaCl₂, 1 mM MgCl₂ and 0.1% gelatin and maintained on ice until assayed. For antagonist studies, aliquots (2 mL) of cells were prewarmed at 37° C for 5 min. in 3 mL plastic cuvettes and fluorescence measured in a fluorometer (Johnson Foundation Biomedical Group, Philadelphia, PA, USA) with magnetic stirring and temperature maintained at 37° C. Excitation was set at 340 nm and emission set at 510 nm. Various concentrations of antagonists or vehicle were added and fluorescence monitored for ∼15 sec to ensure that there was no change in baseline fluorescence, followed by the addition of 33 nM RANTES. Maximal Ca²⁺ attained after 33 nM RANTES stimulation was calculated as described by Grynkiewicz *et al*., (1985). The percent of maximal RANTES-induced Ca²⁺ was determined for each concentration of antagonist and the IC₅₀, defined as the concentration of test compound that inhibits 50% of the maximal 33 nM RANTES response, obtained from the concentration-response curves (5-7 concentrations of antagonists).

The compounds of this invention show CCR5 receptor modulator activity having IC₅₀ values in the range of 0.0001 to 100 µM. The full structure/activity relationship has not yet been established for the compounds of this invention. However, given the disclosure herein, one of ordinary skill in the art can utilize the present assays in order to determine which compounds of formula (I) are modulators of the CCR5 receptor and which bind thereto with an IC₅₀ value in the range of 0.0001 to 100 µM.

The above description fully discloses the invention including preferred embodiments thereof. The embodiments of the invention in which an exclusive property or privilege is claimed are defined as follows.

## Claims

1. The use of a compound selected from:
N-[3-[2-[Bis(1-methylethyl)amino]ethoxy]-4-methoxyphenyl]-2'-ethyl-1,1'-biphenyl-4-carboxamide;
N-[3-[2-[Bis(1-methylethyl)amino]ethoxy]-4-methoxyphenyl]-2'-methoxy-1,1'-biphenyl-4-carboxamide;
N-[3-[2-[Bis(1-methylethyl)amino]ethoxy]-4-methoxyphenyl]-2',6'-dimethyl-1,1'-biphenyl-4-carboxamide;
N-[3-[2-[Bis(1-methylethyl)amino]ethoxy]-4-methoxyphenyl]-3'-ethoxycarbonyl-1,1'-biphenyl-4-carboxamide;
N-[3-[2-[Bis(1-methylethyl)amino]ethoxy]-4-methoxyphenyl]-2',6'-dichloro-1,1'-biphenyl-4-carboxamide;
N-[3-[2-[Bis(1-methylethyl)amino]ethoxy]-4-methoxyphenyl]-2'-chloro-1,1'-biphenyl-4-carboxamide;
N-[3-[2-[Bis(1-methylethyl)amino]ethoxy]-4-methoxyphenyl]-2',4'-dichloro-1,1'-bipheyl-4-carboxamide;
N-[3-[2-[Bis(1-methylethyl)amino]ethoxy]-4-methoxyphenyl]-3'-acetyl-1,1'-biphenyl-4-carboxamide trifluoroacetate;
N-[3-[2-[Bis(1-methylethyl)amino]ethoxy]-4-methoxyphenyl]-3'-methylthio-1,1'-biphenyl-4-carboxamide;
N-[3-[2-[Bis(1-methylethyl)amino]ethoxy]-4-methoxyphenyl]-3',5'-dichloro-1,1'-biphenyl-4-carboxamide;
N-[3-[2-[Bis(1-methylethyl)amino]ethoxy]-4-methoxyphenyl]-3'-isopropoxycarbonyl-1,1'-biphenyl-4-carboxamide; and
N-[3-[2-[Bis(1-methylethyl)amino]ethoxy]-4-methoxyphenyl]-3',5'-bis(methoxycarbonyl)-1,1'-biphenyl-4-carboxamide;
or a pharmaceutically acceptable salt thereof;
in the manufacture of a medicament for the treatment of a CCR5-mediated disease state in mammals wherein the disease is selected from COPD, asthma and atopic disorders, rheumatoid arthritis, sarcoidosis and other fibrotic diseases, atherosclerosis, psoriasis, autoimmune diseases such as multiple sclerosis, inflammatory bowel disease, and HIV infection.

2. A compound selected from the group consisting of:
N-[3-[2-[Bis(1-methylethyl)amino]ethoxy]-4-methoxyphenyl]-2'-ethyl-1,1'-biphenyl-4-carboxamide;
N-[3-[2-[Bis(1-methylethyl)amino]ethoxy]-4-methoxyphenyl]-2'-methoxy-1,1'-biphenyl-4-carboxamide;
N-[3-[2-[Bis(1-methylethyl)amino]ethoxy]-4-methoxyphenyl]-2',6'-dimethyl-1,1'-biphenyl-4-carboxamide;
N-[3-[2-[Bis(1-methylethyl)amino]ethoxy]-4-methoxyphenyl]-3'-ethoxycarbonyl-1,1'-biphenyl-4-carboxamide;
N-[3-[2-[Bis(1-methylethyl)amino]ethoxy]-4-methoxyphenyl]-2',6'-dichloro-1,1'-biphenyl-4-carboxamide;
N-[3-[2-[Bis(1-methylethyl)amino]ethoxy]-4-methoxyphenyl]-2'-chloro-1,1'-biphenyl-4-carboxamide;
N-[3-[2-[Bis(1-methylethyl)amino]ethoxy]-4-methoxyphenyl]-2',4'-dichloro-1,1'-bipheyl-4-carboxamide;
N-[3-[2-[Bis(1-methylethyl)amino]ethoxy]-4-methoxyphenyl]-3'-acetyl-1,1'-biphenyl-4-carboxamide trifluoroacetate;
N-[3-[2-[Bis(1-methylethyl)amino]ethoxy]-4-methoxyphenyl]-3'-methylthio-1,1'-biphenyl-4-carboxamide;
N-[3-[2-[Bis(1-methylethyl)amino]ethoxy]-4-methoxyphenyl]-3',5'-dichloro-1,1'-biphenyl-4-carboxamide;
N-[3-[2-[Bis(1-methylethyl)amino]ethoxy]-4-methoxyphenyl]-3'-isopropoxycarbonyl-1,1'-biphenyl-4-carboxamide; and
N-[3-[2-[Bis(1-methylethyl)amino]ethoxy]-4-methoxyphenyl]-3',5'-bis(methoxycarbonyl)-1,1'-biphenyl-4-carboxamide;
or a pharmaceutically acceptable salt thereof.

3. A phamaceutical formulation comprising a compound as defined in claim 3 and a pharmaceutically acceptable carrier.

## Patentansprüche

1. Verwendung einer Verbindung, die aus folgenden ausgewählt ist:
N-[3-[2-[Bis(1-methylethyl)amino]ethoxy]-4-methoxyphenyl]-2'-ethyl-1,1'-biphenyl-4-carboxamid;
N-[3-[2-[Bis(1-methylethyl)amino]ethoxy]-4-methoxyphenyl]-2'-methoxy-1,1'-biphenyl-4-carboxamid;
N-[3-[2-[Bis(1-methylethyl)amino]ethoxy]-4-methoxyphenyl]-2',6'-dimethyl-1,1'-biphenyl-4-carboxamid;
N-[3-[2-[Bis(1-methylethyl)amino]ethoxy]-4-methoxyphenyl]-3'-ethoxycarbonyl-1,1'-biphenyl-4-carboxamid;
N-[3-[2-[Bis(1-methylethyl)amino]ethoxy]-4-methoxyphenyl]-2',6'-dichlor-1,1'-biphenyl-4-carboxamid;
N-[3-[2-[Bis(1-methylethyl)amino]ethoxy]-4-methoxyphenyl]-2'-chlor-1,1'-biphenyl-4-carboxamid;
N-[3-[2-[Bis(1-methylethyl)amino]ethoxy]-4-methoxyphenyl]-2'4'-dichlor-1,1'-biphenyl-4-carboxamid;
N-[3-[2-[Bis(1-methylethyl)amino]ethoxy]-4-methoxyphenyl]-3'-acetyl-1,1'-biphenyl-4-carboxamidtrifluoracetat;
N-[3-[2-[Bis(1-methylethyl)amino]ethoxy]-4-methoxyphenyl]-3'-methylthio-1,1'-biphenyl-4-carboxamid;
N-[3-[2-[Bis(1-methylethyl)amino]ethoxy]-4-methoxyphenyl]-3',5'-dichlor-1,1'-biphenyl-4-carboxamid;
N-[3-[2-[Bis(1-methylethyl)amino]ethoxy]-4-methoxyphenyl]-3'-isopropoxycarbonyl-1,1'-biphenyl-4-carboxamid; und
N-[3-[2-[Bis(1-methylethyl)amino]ethoxy]-4-methoxyphenyl]-3',5'-bis(methoxycarbonyl)-1,1'biphenyl-4-carboxamid;
oder eines pharmazeutisch akzeptablen Salzes davon;
in der Herstellung eines Medikaments zur Behandlung eines CCR5-vermittelten Krankheitszustandes in Säugetieren, worin die Krankheit ausgewählt ist aus COPD, Asthma und atopischen Störungen, rheumatoider Arthritis, Sarkoidose und anderen fibrotischen Krankheiten, Atherosklerose, Psoriasis, Autoimmunkrankheiten wie multiple Sklerose, entzündlicher Darmerkrankung und HIV-Infektion.

2. Verbindung, die aus der Gruppe ausgewählt ist, die aus folgenden besteht:
N-[3-[2-[Bis(1-methylethyl)amino]ethoxy]-4-methoxyphenyl]-2'-ethyl-1,1'-biphenyl-4-carboxamid;
N-[3-[2-[Bis(1-methylethyl)amino]ethoxy]-4-methoxyphenyl]-2'-methoxy-1,1'-biphenyl-4-carboxamid;
N-[3-[2-[Bis(1-methylethyl)amino]ethoxy]-4-methoxyphenyl]-2',6'-dimethyl-1,1'-biphenyl-4-carboxamid;
N-[3-[2-[Bis(1-methylethyl)amino]ethoxy]-4-methoxyphenyl]-3'-ethoxycarbonyl-1,1'-biphenyl-4-carboxamid;
N-[3-[2-[Bis(1-methylethyl)amino]ethoxy]-4-methoxyphenyl]-2',6'-dichlor-1,1'-biphenyl-4-carboxamid;
N-[3-[2-[Bis(1-methylethyl}amino]ethoxy]-4-methoxyphenyl]-2'-chlor-1,1'-biphenyl-4-carboxamid;
N-[3-[2-[Bis(1-methylethyl)amino]ethoxy]-4-methoxyphenyl]-2'4'-dichlor-1,1'-biphenyl-4-carboxamid;
N-[3-[2-[Bis(1-methylethyl)amino]ethoxy]-4-methoxyphenyl]-3'-acetyl-1,1'-biphenyl-4-carboxamidtrifluoracetat;
N-[3-[2-[Bis(1-methylethyl)amino]ethoxy]-4-methoxyphenyl]-3'-methylthio-1,1'-biphenyl-4-carboxamid;
N-[3-[2-[Bis(1-methylethyl)amino]ethoxy]-4-methoxyphenyl]-3',5'-dichlor-1,1'-biphenyl-4-carboxamid;
N-[3-[2-[Bis(1-methylethyl)amino]ethoxy]-4-methoxyphenyl]-3'-isopropoxycarbonyl-1,1'-biphenyl-4-carboxamid; und
N-[3-[2-[Bis(1-methylethyl)amino]ethoxy]-4-methoxyphenyl]-3',5'-bis(methoxycarbonyl)-1,1'biphenyl-4-carboxamid;
oder ein pharmazeutisch akzeptables Salz davon.

3. Pharmazeutische Formulierung, die eine Verbindung wie in Anspruch 2 definiert und einen pharmazeutisch akzeptablen Träger umfaßt.

## Revendications

1. Utilisation d'un composé choisi parmi :
N-[3-[2-[bis(1-méthyléthyl)amino]éthoxy]-4-méthoxyphényl]-2'-éthyl-1,1'-biphényl-4-carboxamide ;
N-[3-[2-[bis(1-méthyléthyl)amino]éthoxy]-4-méthoxyphényl]-2'-méthoxy-1,1'-biphényl-4-carboxamide ;
N-[3-[2-[bis(1-méthyléthyl)amino]éthoxy]-4-méthoxyphényl]-2',6'-diméthyl-1,1'-biphényl-4-carboxamide ;
N-[3-[2-[bis(1-méthyléthyl)amino]éthoxy]-4-méthoxyphényl]-3'-éthoxycarbonyl-1,1'-biphényl-4-carboxamide ;
N-[3-[2-[bis(1-méthyléthyl)amino]éthoxy]-4-méthoxyphényl]-2',6'-dichloro-1,1'-biphényl-4-carboxamide ;
N-[3-[2-[bis(1-méthyléthyl)amino]éthoxy]-4-méthoxyphényl]-2'-chloro-1,1'-biphényl-4-carboxamide ;
N-[3-[2-[bis(1-méthyléthyl)amino]éthoxy]-4-méthoxyphényl]-2',4'-dichloro-1,1'-biphényl-4-carboxamide ;
N-[3-[2-[bis(1-méthyléthyl)amino]éthoxy]-4-méthoxyphényl]-3'-acétyl-1,1'-biphényl-4-carboxamide trifluoroacétate ;
N-[3-[2-[bis(1-méthyléthyl)amino]éthoxy]-4-méthoxyphényl]-3'-méthylthio-1,1'-biphényl-4-carboxamide ;
N-[3-[2-[bis(1-méthyléthyl)amino]éthoxy]-4-méthoxyphényl]-3',5'-dichloro-1,1'-biphényl-4-carboxamide ;
N-[3-[2-[bis(1-méthyléthyl)amino]éthoxy]-4-méthoxyphényl]-3'-isopropoxycarbonyl-1,1'-biphényl-4-carboxamide ; et
N-[3-[2-[bis(1-méthyléthyl)amino]éthoxy]-4-méthoxyphényl]-3',5'-bis(méthoxycarbonyl)-1,1'-biphényl-4-carboxamide ;
ou d'un sel pharmaceutiquement acceptable de celui-ci ;
dans la fabrication d'un médicament pour le traitement d'un état maladif induite par CCR5 chez les mammifères, dans laquelle la maladie est sélectionnée parmi la bronchopneumopathie chronique obstructive (COPD), l'asthme et des troubles atopiques, la polyarthrite rhumatoïde, la sarcoïdose et autres maladies fibrogènes, l'athérosclérose, le psoriasis, des maladies auto-immunes comme la sclérose en plaques, une affection abdominale inflammatoire et une infection par le VIH.

2. Composé choisi dans l'ensemble consistant en :
N-[3-[2-[bis(1-méthyléthyl)amino]éthoxy]-4-méthoxyphényl]-2'-éthyl-1,1'-biphényl-4-carboxamide ;
N-[3-[2-[bis(1-méthyléthyl)amino]éthoxy]-4-méthoxyphényl]-2'-méthoxy-1,1'-biphényl-4-carboxamide ;
N-[3-[2-[bis(1-méthyléthyl)amino]éthoxy]-4-méthoxyphényl]-2',6'-diméthyl-1,1'-biphényl-4-carboxamide ;
N-[3-[2-[bis(1-méthyléthyl)amino]éthoxy]-4-méthoxyphényl]-3'-éthoxycarbonyl-1,1'-biphényl-4-carboxamide ;
N-[3-[2-[bis(1-méthyléthyl)amino]éthoxy]-4-méthoxyphényl]-2',6'-dichloro-1,1'-biphényl-4-carboxamide ;
N-[3-[2-[bis(1-méthyléthyl)amino]éthoxy]-4-méthoxyphényl]-2'-chloro-1,1'-biphényl-4-carboxamide ;
N-[3-[2-[bis(1-méthyléthyl)amino]éthoxy]-4-méthoxyphényl]-2',4'-dichloro-1,1'-biphényl-4-carboxamide ;
N-[3-[2-[bis(1-méthyléthyl)amino]éthoxy]-4-méthoxyphényl]-3'-acétyl-1,1'-biphényl-4-carboxamide trifluoroacétate ;
N-[3-[2-[bis(1-méthyléthyl)amino]éthoxy]-4-méthoxyphényl]-3'-méthylthio-1,1'-biphényl-4-carboxamide ;
N-[3-[2-[bis(1-méthyléthyl)amino]éthoxy]-4-méthoxyphényl]-3',5'-dichloro-1,1'-biphényl-4-carboxamide ;
N-[3-[2-[bis(1-méthyléthyl)amino]éthoxy]-4-méthoxyphényl]-3'-isopropoxycarbonyl-1,1'-biphényl-4-carboxamide ; et
N-[3-[2-[bis(1-méthyléthyl)amino]éthoxy]-4-méthoxyphényl]-3',5'-bis(méthoxycarbonyl)-1,1'-biphényl-4-carboxamide ;
ou un sel pharmaceutiquement acceptable de celui-ci.

3. Formulation pharmaceutique comprenant un composé tel que défini dans la revendication 3 et un véhicule pharmaceutiquement acceptable.
